**Allied Signal**

Allied Signal Inc.
Law Department
48 Leicester Square
London WC2H 7LW
Telephone 01-839 7022
Telex 21506 UOPINT G
Facsimile 01-930 6412

21 April 1988

European Patent Office
PB 5812 Patent laan 2
2280 HV Rijswijk (ZH)
Netherlands

Dear Sirs

Re:     European Patent Application 88302345.9
        UOP INC (UOP 2116)

With reference to the above-identified application, filed in London on March 17 1988, it is requested that the following clerical errors should be corrected in the specification at an appropriate time:

Page 1 line 20 for "an allyl nickel halide" to read "a $\pi$-allyl nickel halide".

Page 1 line 20 for "and" read "with".

Page 4 line 9 for "diethyl sulphide" read "diethyl disulphide".

Page 10 line 14 for "oligomer" read "olefin".

Page 14, Comparative Example 2 line 4 for "40% n-butene" read "40% n-butane".

Yours faithfully

BROCK Peter William
European Patent Attorney
Allied Signal Inc Law Department

PWB/jb

Europäisches Patentamt

European Patent Office

Office européen des brevets

(19)

(11) Publication number: **0 333 940**

**A1**

(12) # EUROPEAN PATENT APPLICATION

(21) Application number: **88302345.9**

(22) Date of filing: **17.03.88**

(51) Int. Cl.⁴: **C07C 2/30 , B01J 31/28 , C07C 11/02**

A request for correction of pages 1,4,10 and 14 of the ariginally filed description has been filed pursuant to Rule 88 EPC. A decision on the request will be taken during the proceedings before the Examining Division (Guidelines for Examination in the EPO, A-V, 2.2).

(43) Date of publication of application:
**27.09.89 Bulletin 89/39**

(84) Designated Contracting States:
**BE DE ES FR GB GR IT NL**

(71) Applicant: **UOP INC.**
**25 East Algonquin Road**
**Des Plaines Illinois 60017-5017(US)**

(72) Inventor: **Frame, Robert Roy**
**725 Juniper**
**Glenview illinois 60025(US)**
Inventor: **Imai, Tamotsu**
**509 North Eastwood Avenue**
**Mount Prospect Illinois 60056(US)**

(74) Representative: **Brock, Peter William**
**URQUHART-DYKES & LORD 91 Wimpole Street**
**London W1M 8AH(GB)**

(54) **Process and catalyst for the oligomerization of olefins.**

(57) Olefinic feedstocks which contain catalyst contaminants or poisons such as sulphur-containing compounds may be oligomerized with a high selectivity to a desired oligomer and configuration of the oligomer by utilizing a catalyst which is stable and will not deactivate due to the presence of the aforementioned poisons. The catalyst comprises a porous support which has been impregnated with an iron group metal, such as iron or nickel, and a non-transition metal of Group IV of the Periodic Table, such as tin or germanium. In addition, the catalyst also contains an alkyl aluminium compound and, in preferred embodiments, an aluminium alkoxy compound and/or aluminium halide.

EP 0 333 940 A1

# PROCESS AND CATALYST FOR THE OLIGOMERIZATION OF OLEFINS

This invention relates to a novel catalyst system which is specifically designed to oligomerize olefins to form higher molecular weight olefins; for example, selectively to convert propylene into a $C_6$ olefin.

The oligomerization of olefins is known in the art, such oligomerization processes being effected by treating olefinic hydrocarbons with certain catalysts to obtain various oligomers which will find a useful function in the chemical art. One type of catalyst which may be employed for this particular type of reaction comprises a supported metal compound. For example, US-A-3562351 discloses a method for dimerizing olefins utilizing a supported catalyst which has been prepared by impregnating a suitable support with a salt solution of a Group VIII metal, followed by a heat treatment in an inert atmosphere at a temperature less than that which is required to form a metal oxide but which will form a complex on the surface of the solid support. Following this, the catalyst is activated by treatment with an organometallic compound. US-A-3483269 describes a catalyst useful for oligomerizing lower olefins which comprises an allyl nickel halide supported on an acidic inorganic oxide support. If so desired, the support may have been optionally treated with an alkyl aluminium compound. US-A-3592869 also describes a catalyst which is useful for the oligomerization of olefins. A divalent nickel compound and an alkyl aluminium compound are contacted with an olefinic compound. The resulting mixture is then used to impregnate an inorganic refractory oxide support. US-A-3644564, describes a catalyst for the oligomerization of ethylene which comprises an organo aluminium-free reaction product of a nickel compound which is an atom of nickel in complex with an olefinically unsaturated compound and a fluorine-containing ligand. The catalysts are typically formed in situ. US-A-3679772 describes a process for reacting monoolefins with diolefins, the catalyst for such a reaction comprising a complex of (1) nickel, (2) a group VA electron donor ligand, such as an organophosphine, (3) a non-protonic Lewis acid and (4) a reducing agent, which itself may be a Lewis acid, all of which are composited on an acidic silica-based support.

As will hereinafter be shown in greater detail, the oligomerization of olefinic hydrocarbons to provide products which possess a desired configuration with respect to the branching or minimal branching of the resultant chain may be accomplished by treating said olefins in the presence of a catalyst of the present invention, this catalyst will maintain its activity and stability for a relatively long period of time in the presence of certain impurities or poisons in the feedstock.

This invention relates to a catalyst which is useful for the oligomerization of olefinic hydrocarbons. More specifically, the invention is concerned with a catalyst and a process for the oligomerization of olefinic compounds, particularly olefinic hydrocarbons, whereby the use of the catalyst will result in the selective production of oligomers from the olefinic feed stock.

The term "polymerization" has a relatively broad meaning in the chemical art. Although it is generally referred to as the preparation of relatively high molecular weight polymers, that is polymers possessing molecular weights of greater than 50,000, it may also refer to low molecular weight polymers, that is, polymers possessing molecular weights lower than 50,000. In contradistinction to this, the term "oligomerization" refers to the production of polymers whose molecules consist of only a relatively few monomeric units; and this oligomerization would include dimerization, trimerization or tetramerization.

Therefore this invention is concerned with the problem of providing a catalyst for the oligomerization of olefinic hydrocarbons, and more specifically of providing a specific catalyst system which may be used in a process for the oligomerization of olefinic hydrocarbons to provide selective oligomers.

One embodiment of this invention provides a catalyst comprising a combination of an alkyl aluminium compound on a porous support containing an iron group metal, such as nickel or iron, and a non-transition metal of Group IV of the Periodic Table, such as tin or germanium.

Another embodiment of this invention provides a process for the oligomerization of an olefinic hydrocarbon which comprises contacting a charge stock containing said hydrocarbon with a catalyst as defined above.

A specific embodiment of this invention provides a catalyst comprising a combination of diethyl aluminium chloride on an alumina support, containing catalytically nickel and tin, wherein the nickel is preferably present as a hydrated nickel oxide and the tin as stannic chloride.

Another specific embodiment of this invention is a process for the oligomerization of butene in the presence of a catalyst comprising a combination of diethyl aluminium chloride and tri-t-butoxy aluminium on an alumina support which contains a nickel compound and stannic chloride, at a temperature from -20 to 200°C and a pressure of 2.5 to 7 MPa (350 to 1,000 psig), and recovering the resultant oligomer comprising a mixture of octene, methylheptene and dimethylhexene.

The preparation of a catalyst composite which may be used for the polymerization of oligomerization of olefinic compounds was heretofore relatively difficult, inasmuch as several relatively expensive compounds were required as components of the composite, as well as entailing somewhat complicated methods for the manufacture thereof. In contradistinction to this, the catalyst composite of the present invention is relatively easy to prepare and, in addition, includes compounds which are less expensive than the components of the known catalysts. The final catalyst of the present invention will possess a high activity and will be stable over a relatively long period of time. Furthermore, the catalyst of the present invention will possess the desired stability and activity due to its ability to effect the desired oligomerization in spite of the presence of impurities which would, under ordinary circumstances, be expected to poison or deactivate the catalyst.

The particular impurities which are present in the feedstock, and which act to deactivate catalysts, comprise various sulphur compounds such as carbonyl sulphide or hydrogen sulphide; mercaptans such as methyl mercaptan, ethyl mercaptan or propyl mercaptan; and disulphides such as dimethyl disulphide or diethyl sulphide. In addition, other catalyst-deactivating impurities include oxygenates comprising oxygen-containing organic compounds such as alcohols, including methyl alcohol, ethyl alcohol and propyl alcohol; ethers such as dimethyl ether, diethyl ether, methyl ethyl ether and methyl propyl ether; aldehydes such as formaldehyde and acetaldehyde; and ketones such as acetone and methyl ethyl ketone. Generally speaking, the impurities may be present in trace amounts, such as in a range of from 0.1 to 100 ppm of feedstock. However, even these trace amounts of impurities will have a deleterious effect upon the activity and stability of oligomerization catalysts, and thus quickly deactivate the catalyst when they are present.

It has now unexpectedly been discovered that the novel catalysts of the present invention, which are prepared according to the methods hereinafter set forth, will exhibit an unexpected resistance to the poisoning effect of the impurities and thus, will permit the catalyst to maintain a desired activity, as well as being stable for a long period of time when employed in the conversion of olefinic hydrocarbons into desired dimers. In addition to these desired attributes, the catalyst will also produce a high yield of dimer products, especially from $C_3$ and $C_4$ olefins, as compared to trimer and tetramer products. The dimer products produced by the oligomerization of propylene or the n-butenes will possess a high percentage of linear compounds, that is, n-hexenes and n-octenes, and also a high percentage of dimers which contain only one methyl substituent; more highly branched oligomers being minority products. The propylene dimers which are produced by the process of the present invention all possess high octane numbers regardless of the branching, and thus are excellent octane blending components. In addition, the n-butene dimers are excellent as intermediates in the preparation of plasticizers.

The catalyst composite of the present invention comprises a combination of a catalytically effective amount of an alkyl aluminium compound with a calcined porous support which, before calcination, contained a hydrate of an iron group metal salt, and a compound containing a non-transition metal of Group IV of the Periodic Table. In the preferred embodiment of the invention, the iron group metal hydrate will be obtained from a soluble hydrated compound of nickel, iron or cobalt, for example, nickel nitrate, nickel hydroxide, nickel bromide, nickel chloride, nickel fluoride, nickel acetate, cobaltic chloride, cobaltous acetate, cobaltous ammonium chloride, cobaltous bromide, cobaltous fluoride, cobaltous perchlorate, cobaltous sulphate, ferrous acetate, ferrous or ferric bromide, and ferrous or ferric chloride. The compound containing the non-transition metal of Group IV (referred to hereinafter as members of Group IVA of the Periodic Table) will comprise salts of these metals, such as germanium chloride, germanium iodide, germanium fluoride or germanium sulphide; or stannic bromide, stannic chloride, stannic oxychloride, stannic sulphate, lead acetate or lead perchlorate. It is also contemplated within the scope of this invention that these metals may also be used in their elemental form as one component of the catalyst of the present invention.

The porous support which is impregnated with the iron group metal hydrate can be an inorganic metal oxide such as alumina or silica; mixtures of oxides such as alumina-silica or alumina-zirconia-magnesia; or crystalline aluminosilicates which are commonly known as zeolites.

Examples of alkyl aluminium compounds which form another component of the catalyst will include alkyl aluminium halides such as dimethyl aluminium chloride, diethyl aluminium chloride, dipropyl aluminium chloride, dimethyl aluminium bromide, diethyl aluminium bromide, dipropyl aluminium bromide, dimethyl aluminium iodide, diethyl aluminium iodide or dipropyl aluminium iodide.

One optional component, which may be present in the catalyst composite of the present invention, will comprise an aluminium alkoxy compound which will act as an activator for the catalytic composite. Examples of these aluminium alkoxy compounds will have the general formula $Al(OR)_3$ in which each R is lower alkyl having from 1 to 6 carbon atoms. Some specific examples of these aluminium alkoxy compounds include trimethoxy aluminium, triethoxy aluminium, tripropoxy aluminium, triisopropoxy aluminium, tri-n-butoxy aluminium, tri-t-butoxy aluminium, tripentoxy aluminium and trihexoxy aluminium. It is also contemplated within the scope of this invention that the catalyst may, if so desired, also contain an aluminium halide in place of the aluminium alkoxy compound or in conjunction therewith. Examples of these

3

aluminium halides include aluminium chloride, aluminium bromide and aluminium iodide. It is to be understood that the aforementioned list of iron group metal compounds, Group IVA metal compounds, alkyl aluminium compounds, and aluminium alkoxy compounds, are only representative of the class of compounds which may be employed to form the catalyst composite of the present invention.

The oligomerization catalyst of the present invention may be prepared in such a manner as to provide the finished catalyst with the desired characteristics with regard to the selectivity of olefins obtained by the reaction of an olefinic hydrocarbon in the presence of such catalysts, as well as specificity of the product so obtained. The catalyst composite may be prepared by impregnating a porous support of the type hereinbefore set forth with a simple divalent iron group metal salt for example, nickel nitrate, and a Group IVA metal salt, such as tin chloride, preferably from an aqueous solution. Alternatively, the porous support may be impregnated with an aqueous iron group metal salt and the Group IVA metal may be incorporated into the support by any techniques which are well known to those skilled in the art. For example, the support may be impregnated with sols such as tin or germanium sols by well known sol-gel techniques. In any event, after impregnation of the porous support, such as alumina, which can be effected at ambient temperature and atmospheric pressure, the impregnated support is then subjected to a thermal treatment. By varying the temperature of the thermal treatment, it is possible to obtain a catalyst composite which will provide a greater selectivity to dimer products, which will be found to be present in greater amounts, in contrast to trimer and tetramer products, than are obtained when using conventional oligomerization catalysts. The thermal treatment of the impregnated support is preferably effected at a temperature of from 300 to 450°C, the preferred temperature being from 340 to 360°C. The thermal treatment or calcination of the catalyst base containing the impregnated Group IVA metal or salt thereof, and iron group metal salt in hydrate form, will result in a weight loss due to a loss of water of hydration from the metal salt and will result in the formation of a non-stoichiometric hydrogen- and oxygen-containing iron group metal compound which may also be referred to as a hydrate of an iron group metal oxide and/or salt. In the preferred embodiment of the invention, the mole ratio of water of hydration to iron group metal following the thermal treatment will be greater than 0.5:1, and preferably be a range from 0.5:1 to 6:1. The thermal treatment of the catalyst base containing the iron group metal compound in the form of a hydrate will usually be effected for a period of time which is less than that required completely to drive off all the water of hydration.

The thermal treatment or calcination of the catalyst base and the iron group metal salt at temperatures within the range hereinbefore set forth will result in a bonding of the iron group metal to the catalyst base, usually by means of a metal-oxygen-base bond, the oxygen portion of the bond being supplied in part by the hydroxyl groups which are present on the surface of the porous support of the type hereinbefore set forth in greater detail.

Following the thermal treatment, the iron group metal-impregnated catalyst base is then treated with an alkyl aluminium compound, and optionally with an aluminium alkoxy compound or an aluminium halide to produce a catalyst of maximum activity. The treatment of the base with the aluminium alkyl compound and the activator is also effected at ambient temperature and atmospheric pressure, utilizing a solution of the treating compound or compounds dissolved in an organic solvent, such as benzene, toluene or xylene isomers.

The addition of the organic solution, or conversely the addition of the calcined base to the organic solution, will result in an exothermic reaction and, after thorough mixing, the resulting solution containing the impregnated base is allowed to return to room temperature. The solvent may then be removed by conventional means, such as decantation, evaporation, or filtration, and the catalyst composite may then be washed with an organic solvent to remove residues or traces of unwanted compounds. Thereafter, the catalyst may then be dried by purging with nitrogen, and recovered. The alkyl aluminium compound is preferrably present in the finished composite in a mole ratio of from 0.05:1 to 6:1, more preferrably from 0.1:1 to 1:1, moles of alkyl aluminium compound per mole of iron group metal, the latter preferrably being present in said composite, on an elemental basis, in an amount from 1 to 20% by weight of the composite, and more preferrably in an amount from 1 to 10% by weight. In addition, the Group IVA metal will also be present in said composite, on an elemental basis, in an amount of preferrably from 0.1 to 20% by weight of the composite, and more preferrably in an amount from 1 to 10%. The weight ratio of Group IVA metal to iron group metal is preferrably maintained in the range from 0.1:1 to 10:1 for best results.

As will hereinafter be shown in greater detail, by preparing a catalyst which possesses the various components in the finished composite in mole ratios or weight percent ages within the ranges hereinbefore set forth, it is possible selectively to oligomerize olefin compounds containing from 2 to 6 carbon atoms, obtaining desirable isomers in each of the oligomer products. In addition, by utilizing a metal of the Group IVA of the Periodic Table, as well as an aluminium alkoxide compound, it is possible to obtain a catalyst composite which will be more stable, in that it will not deactivate in the presence of the type of impurities hereinbefore set forth in greater detail, which may themselves be present in the feedstock, and which may impede or deter the oligomerization reaction.

As an example of how the catalyst composite of the present invention may be prepared, a predetermined amount of a porous base, such as alumina, silica, silica-alumina or an aluminosilicate, which may be in the form of spheres, pellets or rods, may be prepared in an appropriate apparatus, such as an evaporator, along with an aqueous solution of a hydrate of an iron group metal salt and a Group IVA metal salt.

The apparatus may be heated, after thorough mixing, to form the desired iron group metal- and Group IVA metal-impregnated base. The impregnated base may then be placed in a heating apparatus, such as a tube furnace, and treated with air while bringing the catalyst to a temperature of about 250°C. The heating is accomplished at a relatively slow rate, and after the determined temperature has been reached, it is maintained thereat for an additional period which may be from 2 to 4 hours, or even longer. The calcination of the catalyst base is then effected by increasing the temperature to a predetermined level, and maintaining thereat for a period sufficient to bring the mole ratio of water of hydration present in the iron group metal salt to a determined level, which is preferrably in an excess of about 0.5:1 moles of water of hydration per mole of iron group metal.

After allowing the calcination to proceed for this predetermined period, heating is discontinued and the catalyst base which contains from 1 to 20% by weight of Group IVA metal, is allowed to cool. The cooled base may then be mixed with a solution of an alkyl aluminium compound and an aluminium halide/aluminium alkoxy compound dissolved in an organic solvent. As previously, discussed, the resulting reaction is exothermic in nature and after allowing the heat to dissipate, the resulting mixture is thoroughly stirred and allowed to stand for a period which may be from 1 to 100 hours, or even more. At the end of this period, the organic solvent is removed by decantation, filtration, centrifugation, etc and the solid catalyst is washed to remove any unreacted material. After washing, the catalyst is dried in an inert atmosphere, such as that provided by the presence of nitrogen, and recovered.

The oligomerization of olefins containing from 2 to 6 carbon atoms, such as ethylene, propylene, butene-1, butene-2, pentene-1, pentene-2 or pentene-3, may then be effected by treating the oligomer in the presence of the catalyst at oligomerization conditions which will generally include a temperature of from -20 to 200°C, the preferred range being from 30 to 100°C, and a pressure of from 2.5 MPa to 7 MPa (350 to 1,000 psig). The pressure which is utilized may be the autogenous pressure provided for by the feedstock, if in gaseous phase, or the feedstock may supply only a partial pressure, the remainder of said pressure being provided by the introduction of an inert gas such as nitrogen, helium, or argon, into the reaction zone.

It is contemplated within the scope of this invention that the oligomerization process may be effected in either a batchwise or continuous operation. For example, when a batchwise operation is employed, a quantity of the novel catalyst of the present invention may be placed in an appropriate apparatus for example, a rotating, mixing or stirring autoclave. If the olefinic feedstock is in gaseous form, the autoclave is sealed and the feedstock comprising the olefinic hydrocarbon or a mixture of olefinic and paraffinic hydrocarbon of similar carbon chain length, is charged to the reactor until the desired operating pressure has been attained. The apparatus is then heated to the desired operating temperature and maintained thereat for a predetermined period which may be from 1 to 6 hours, or longer. At the end of this period, heating is discontinued, and after the apparatus and its contents have returned to room temperature, the excess pressure is discharged and the autoclave is opened. The reaction product is recovered, separated from the catalyst by conventional means, such as decantation, filtration, or centrifugation, and, if so desired, subjected to fractional distillation, whereby the various isomers may be separated from one another, and stored. Conversely, if so desired, the reaction product comprising a mixture of isomers may be recovered and stored per se without separating the various isomer fractions which are present in the product mixture.

In the event that the olefinic charge stock is in liquid form, it may be charged to the reactor which is thereafter sealed and pressurized to the desired operating pressure by the introduction of an inert gas of the type hereinbefore set forth. The remainder of the operating pressure to obtain the desired oligomer product is carried out in a manner similar to that previously described.

When utilizing continuous operation to obtain the desired oligomer products, a quantity of the catalyst composite is placed in an appropriate apparatus. The feedstock comprising the olefinic compound is continuously charged to this reactor which is maintained at the proper operating temperature and pressure. As in the case of the batchwise operation, the desired operating pressure may be provided for by the olefinic hydrocarbon itself, or by the addition of a heated inert gas. After passage through the reactor for a predetermined period, the reactor effluent is continuously discharged and the reaction product may be recovered and passed to storage, or it may be passed to a distillation apparatus whereby separation of the

5

various isomers and oligomers may be effected. Any unreacted olefinic hydrocarbon recovered from the reactor effluent may be recycled back to the reactor to form a portion of the feed charge.

Inasmuch as the catalyst composite of the present invention is in solid form, the continuous method of operation for obtaining the desired oligomers may be effected in various ways. For example, the catalyst may be positioned as a fixed bed in the reaction zone and the olefinic feedstock may be passed over the catalyst bed in either an upward or downward flow. Another type of continuous operation, is a moving bed operation in which the catalyst bed and the feedstock are passed through the reaction zone, either concurrently or countercurrently to each other. In addition to the fixed or moving bed type of operation, it is also contemplated that the slurry type of operation may be employed. Especially when the olefinic hydrocarbon feedstock is in liquid form. When this type of operation is employed, the catalyst is charged to the reactor as a slurry in the olefinic feedstock.

Examples of oligomers of olefinic compounds which may be obtained when utilizing the catalyst composite of the present invention will include n-butene, isobutene, n-hexene, methylpentene, dimethylbutene, n-octene, the isomeric heptenes, dimethyl hexenes, n-dodecene, the isomeric methyl undecenes, dimethyl decenes, etc. As was previously stated, the oligomer products which are obtained in the process of this invention will comprise, in the main, the dimers of the particular olefinic compound which was employed as the feedstock, thus, for example, when employing ethylene as the feed, the reaction product will comprise mostly $C_4$ olefins; when employing propylene as the feedstock, the reaction product will comprise mostly $C_6$ olefins; and when employing butene as the feedstock, the reaction product will comprise mostly $C_8$ olefins. Thus, the catalyst composite of the present invention will result in products which find particular uses in the finished product.

The following examples are given for purposes of illustrating the novel catalyst composites of the present invention, methods for preparing these composites and a process for utilizing these composites.

## COMPARATIVE EXAMPLE I

A control catalyst was prepared by impregnating 250 cc of 3 mm (1/8 inch) alumina spheres with an aqueous solution of nickel nitrate hexahydrate. The impregnation was effected in a steam-jacketed rotary evaporator in which the mixture was rolled for 0.5 hours at ambient temperature. The evaporator was then heated with steam for 2 hours, during which time the water phase was evaporated. Following this, the nickel nitrate-impregnated alumina base was loaded into a tube furnace and a flow of air was established, the air passing through the catalyst bed at a rate of 600 cc per minute. The catalyst was brought to a temperature of 250°C and maintained thereat for 3 hours. Thereafter, the temperature was increased to 400°C and maintained thereat for an additional 2 hours. Following this, heating was discontinued and the impregnated catalyst base, containing 5.5 wt. % of nickel, was recovered.

After cooling the impregnated alumina, it was then activated by adding 7.3 grams of diethyl aluminium chloride and 2.9 grams of tri-sec-butoxy aluminium per 100 cc of support, as a toluene solution, the activation of the alumina base being effected in a glove box under a nitrogen atmosphere. The addition of the activator was accomplished by slowly adding the activator solution over 15 minutes to the catalyst base, heat being evolved during this addition due to the exothermic nature of the reaction. After completion of the addition of the activator solution, the flask was intermittently swirled over 12 hours. At the end of the 12 hour period, the solvents were decanted and the catalyst composite was washed with 6 portions of isopentane, utilizing 100-115 cc per wash. The resulting catalyst composite was then allowed to dry by evaporation of the excess isopentane, while maintaining an atmosphere of nitrogen in the glove box.

## EXAMPLE 1

In this example, a catalyst composite of the present invention was prepared by impregnating a catalyst base of 3 mm (1/8 inch) spheres with an aqueous solution of nickel nitrate hexahydrate and stannic chloride pentahydrate, the impregnation of the base with the aqueous solution being accomplished in a manner similar to that hereinbefore set forth in Comparative Example 1. The water present was evaporated by use of a steam-jacketed rotary evaporator.

The nickel nitrate/tin chloride-containing alumina base was placed in a tube furnace under a flow of air and calcined at a temperature of 250°C for 3 hours, followed by a calcination at 400°C for 2 hours. The finished calcined support contained 4.5 wt. % nickel and 1.8 wt. % tin after calcination.

The activation of the catalyst base was effected by placing said base covered with toluene in an Erlenmeyer flask in a glove box, a toluene solution of diethyl aluminium chloride and tri-sec-butoxy aluminium was slowly added over 15 minutes to prevent overheating of the catalyst base due to evolution of heat in the exothermic reaction. The weights of the activating compounds per 100 cc of support were 2.9 grams of tri-sec-butoxy aluminium and 7.3 grams of diethyl aluminium chloride. After intermittently swirling the flask during a period of 12 hours, the toluene was decanted and the catalyst composite was washed with 6 portions of isopentane. Thereafter, it was allowed to dry in a glove box under a nitrogen atmosphere until it was free-flowing in nature.

## COMPARATIVE EXAMPLE 2

The control catalyst which was prepared in accordance with Comparative Example 1 above was utilized in the oligomerization of a butene feedstock comprising a mixture of 60% butene and 40% n-butene which also contained 30 ppm by weight of dimethyl disulphide and 45 ppm by weight of methyl t-butyl ether. The oligomerization was effected by placing 50 cc of the catalyst in a tubular reactor having a 12.5 mm ($\frac{1}{2}$ inch) inner diameter. The feedstock was charged downflow to the reactor at an LHSV of 2.0 hour$^{-1}$ while maintaining reaction conditions which included an inlet temperature of 70°C and a pressure of 4.9 MPa (700 psig). Feedstock was charged to the reactor only for a period of 12 hours. During this time the catalyst bed maximum temperature was never more than 1.3°C above the inlet temperature. Oligomerization is a strongly exothermic reaction and during runs wherein high n-butene conversions are observed, much higher catalyst bed maximum temperatures are observed. The average n-butene conversion over the 12 hour run, calculated from the n-butene/n-butane compositions of the feed and the product was only 10 wt. % (Table 1).

Only a small amount of C$_4$$_+$ liquid was collected. It had the properties set out in the Table below.

By the end of 12 hours, the small exotherm had moved down the bed, as is characteristic of a catalyst deactivation due to a feed poison.

## EXAMPLE 2

To illustrate the efficacy of the catalyst of the present invention, in which the alumina base was impregnated with both nickel and tin, with regard to its ability to remain stable and active in the presence of impurities which normally act as catalyst poisons, a second run was made. In this run, 50 cc of the catalyst of the present invention, which was prepared according to Example 1 above, were placed in a tubular reactor similar to that described in the Comparative Example 2. The conditions which were employed in this oligomerization reaction were similar to those utilized in Comparative Example 2, that is, a feedstock of 60% butene and 40% n-butane which contains 30 ppm by weight of dimethyl disulphide and 45 ppm by weight of methyl t-butyl ether, was charged to the reactor at an LHSV of 2.0 hrs$^{-1}$, while maintaining an inlet temperature of 70°C and a pressure of 4.9 MPa (700 psig). The reaction was allowed to proceed for 92 hours, samples being taken intermittently during this period and analyzed by gas chromatography. The results of these analyses are set forth in the Table below:

7

TABLE

| Catalyst | Hours | Butene Conversion % | Selectivity C₈ (wt. %) | C₈ Isomer Distribution % | | |
|---|---|---|---|---|---|---|
| | | | | n-Octene | Methyl Heptene | Dimethyl Hexene |
| Example 2 | 0-12 | 71.0 | 84.4 | 10.4 | 64.7 | 24.9 |
| | 13-24 | 67.4 | 85.8 | 10.6 | 64.9 | 24.5 |
| | 25-36 | 62.5 | 87.3 | 11.5 | 64.5 | 24.0 |
| | 37-48 | 48.9 | 86.6 | 12.0 | 64.7 | 23.3 |
| | 61-72 | 38.1 | 87.2 | 13.4 | 65.0 | 21.6 |
| Comparative Example 2 | 0-12 | 10.0 | 89.2 | 10.9 | 64.6 | 24.5 |

It is evident from a comparison of the results obtained, that by utilizing a catalyst of the present invention as compared to a catalyst which does not contain a Group IVA metal e.g. results in superior performance with regard to stability and activity.

## Claims

1. An olefin-oligomerization catalyst characterized in that it comprises a porous support, an alkyl aluminium compound, an iron group metal, and a nontransition metal of Group IV of the Periodic Table.

2. A catalyst according to Claim 1 characterized in that it additionally contains an aluminium alkoxy compound.

3. A catalyst according to Claim 1 or 2 characterized in that it additionally contains an aluminium halide.

4. A catalyst according to any one of Claims 1 to 3 characterized in that it contains, on an elemental basis, from 1 to 20% by weight of iron group metal, the mole ratio of alkyl aluminium compound to iron group metal being from 0.5:1 to 6:1 moles, and the weight ratio of Group IV metal to iron group metal being from 0.1:1 to 10:1.

5. A catalyst according to any one of Claims 1 to 4 characterized in that the iron group metal is iron or nickel, and the Group IV metal is tin or germanium.

6. A process for the oligomerization of an olefinic hydrocarbon which comprises contacting a charge stock containing said hydrocarbon at oligomerization conditions with a catalyst, characterized in that the catalyst is as defined in any one of Claims 1 to 5.

7. A process according to claim 6 characterized in that said oligomerization conditions include a temperature from -20°C to 200°C and a pressure 2.5 MPa to 7 MPa (350 to 1000 Psig).

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A-1 123 474 (BRITISH PETROLEUM CO.) * page 1, lines 28-44; claims 1,2,4-11,13 * | 1-7 | C 07 C 2/30 B 01 J 31/28 C 07 C 11/02 |
| Y | EP-A-0 202 670 (VOP INC.) * claims 1-11 * | 1-7 | |
| A | US-A-4 013 738 (POLLITZER et al.) * abstract; claims 1,4-6,13 * | 1 | |
| A | FR-A-2 047 214 (ESSO RESEARCH & ENGINEERING CO.) * claims 1,2,4,6,7,9,13-16; page 11, lines 10-15 * | 1,2,5,7 | |
| A,D | US-A-3 592 869 (L.G. CANNELL et al.) * abstract; claims 1,3-6,8,10 * | 1,3,5,7 | |
| A | US-A-3 530 200 (P.W. GLOCKNER et al.) * abstract; claims 1-3,8-10 * | 1,5 | |
| A | US-A-3 168 590 (J.R. KENTON et al.) * claims 1,2 * | 1,7 | TECHNICAL FIELDS SEARCHED (Int. Cl.4) C 07 C 2/00 B 01 J 31/00 C 07 C 11/00 |
| A | CHEMICAL ABSTRACTS volume 77, no. 13, 25th September 1972, Columbus, Ohio, USA, page 39, column 2, abstract no. 87808f; & JP - A - 7 222 206 (JAPAN SYNTHETIC RUBBER) 22-06-1972 | 1 | |

The present search report has been drawn up for all claims

| Place of search | Date of completion of the search | Examiner |
|---|---|---|
| BERLIN | 24-10-1988 | RUFET J.M.A. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P0401)